# EUROPÄISCHE PATENTSCHRIFT

(11) **EP 3 501 465 B1**
(45) Veröffentlichungstag und Bekanntmachung des Hinweises auf die Patenterteilung: **26.07.2023**
(21) Anmeldenummer: 18213647.3
(22) Anmeldetag: 18.12.2018
(51) Int. Cl.: A61F 13/02, A61F 13/00, A61L 15/60, B26D 3/28, A61L 15/42, B26D 7/27, B26F 1/38

(54) **VERFAHREN ZUR HERSTELLUNG EINER WUNDAUFLAGE**
METHOD FOR PRODUCING A WOUND DRESSING
PROCÉDÉ DE FABRICATION D'UN PANSEMENT

(30) Priorität: 21.12.2017 DE 102017131013
(43) Veröffentlichungstag der Anmeldung: 26.06.2019
(73) Patentinhaber: Paul Hartmann AG, 89522 Heidenheim (DE)
(72) Erfinder: Frühe, Thomas, 89518 Heidenheim (DE); Ward, James, Salford, M50 3AU (GB); Kemmler, Julia, 89077 Ulm (DE); Junginger, Martin, 89568 Hermaringen (DE); Röttger, Marc, 89129 Langenau (DE); Maier, Fabian, 89198 Westerstetten (DE); Bernd, Frank, 89555 Steinheim (DE)
(74) Vertreter: DREISS Patentanwälte PartG mbB

(56) Entgegenhaltungen:
- EP-A1- 2 158 924
- WO-A1-2013/009239
- DE-A1- 10 212 866
- US-A- 5 133 821
- US-A1- 2003 120 229
- US-B1- 6 309 500

## Beschreibung

Die Erfindung betrifft ein Verfahren zur Herstellung einer mehrlagigen Wundauflage unter Verwendung eines für medizinische Zwecke geeigneten Laminats. Die Erfindung betrifft außerdem eine mehrlagige Wundauflage, die unter Verwendung eines für medizinische Zwecke geeigneten Laminats hergestellt ist.

Ein Laminat, das für medizinische Zwecke geeignet ist, ist im vorliegenden Sinne ein flächiges und mehrschichtiges Material mit einer ersten Außenseite und einer zweiten Außenseite, wobei das Laminat mindestens eine poröse Lage sowie eine Wundkontakt-Lage, die sich von der porösen Lage unterscheidet, aufweist. Unter der Bezeichnung "für medizinische Zwecke geeignet" wird im vorliegenden Zusammenhang verstanden, dass das Laminat ausschließlich physiologisch verträgliche Materialien enthält, die für eine Wundbehandlung geeignet sind.

Die Erfindung betrifft insbesondere Wundauflagen und deren Herstellung, die geeignet sind, um zur feuchten Wundbehandlung eingesetzt werden können, sowie Verfahren zu deren Herstellung.

Bei der Wundbehandlung besteht ein Bedürfnis nach Wundauflagen, welche die Heilung von Wunden beschleunigen und den natürlichen Wundheilungsprozess unterstützen. Wundauflagen sollen dabei häufig unterschiedliche Eigenschaften in Kombination aufweisen.

Die Heilung von Hautwunden beruht auf der Fähigkeit der Haut Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben.

Bei der Heilung von Hautwunden werden häufig drei wesentliche Heilungsphasen einer Wunde unterschieden, die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungs-phase) .

Zur Unterstützung der einzelnen Wundheilungsphasen sind verschiedene Wundauflagen in der Literatur beschrieben.

In WO 02/ 38 097 A1, WO 02/ 47 761 A1, WO 03/ 011 352 A1, WO 03/ 086 255 A1, WO 2004/ 052 415 A1 oder EP 1 658 865 A1 werden Wundauflagen beschrieben, die ein Hydrogel und einen Polymerschaum umfassen. In WO 2010/000 450 des Anmelders Paul Hartmann AG, ist gleichfalls eine mehrschichtige bzw. mehrlagige Wundauflage mit einer Wundkontaktschicht als erster Schicht und mindestens einer zweiten Schicht als absorbierende Schicht, die einen hydrophilen Polyurethanschaum umfasst, beschrieben. Die Wundkontaktschicht kann ein Hydrogel auf Basis eines Polyharnstoff/Polyurethan-Copolymers sein.

Wundauflagen zur Behandlung chronischer Wunden werden typischerweise derart appliziert, dass der Rand der Wundauflage auf der die Wunde umgebenden, gesunden Haut zu liegen kommt. Beispielsweise werden Schaumwundauflagen, welche in Verbindung mit der Kompressionstherapie eingesetzt werden, auf diese Art appliziert. Die Wundauflage wird den Wundrand überlappend appliziert und mittels einer unter Druck gewickelten Binde fixiert. Durch die Auflage des Randes der Wundauflage auf der Haut kann sich für die in der Umgebung der Wunde vorhandene Haut eine erhebliche Belastung ergeben.

Wundauflagen mit abgeflachten Rändern können eingesetzt werden, um diese Belastungen zu mindern.

Schaumstoff-Wundauflagen, welche abgeflachte Ränder aufweisen, können im einfachsten Fall durch ein Beschneiden des Randes oder durch Gießen des noch nicht ausgehärteten Schaumes in eine entsprechend ausgestaltete Gießform erhalten werden, wie in WO91/01706 erwähnt.

Eine Möglichkeit zur Herstellung von Wundauflagen, welche abgeflachte Ränder aufweisen, ist in EP 1 931 289 B1 beschrieben. Demnach können abgeflachte Ränder erhalten werden, indem ein nur teilweise ausgehärteter Schaum randständig zusammengepresst wird. Der im Randbereich vorhandene Schaum weißt dann im Vergleich zum zentralen Bereich der Wundauflage eine erhöhte Dichte auf. US 5 133 821 A offenbart ein Verfahren zur Konturierung einer Hydrokolloid-Wundauflage.WO 2013/009239 A1 offenbart ein Verfahren zur Herstellung eines selbstklebenden Wundpflegeproduktes, bei dem ein Werkzeug mit einer abgeschrägten Pressfläche und Kante benutzt wird.DE 102 12 866 A1 offenbart ein Narbenreduktionspflaster und ein Verfahren zu Herstellung, bei dem mit einer Walze eine gewünschte Form aufgewalzt wird.US 6 309 500 B1 offenbart ein Verfahren zur Herstellung einer profilierten Wundauflage.US 2003/120 229 A1 offenbart ein Herstellungsverfahren von mehrschichtigen Wundauflagen.EP 2 158 924 A1 offenbart ein Verfahren zur Herstellung eines geformten Artikels mit einer Schaumschicht, die mittels Temperatur und Druck verformt wird.

Die Möglichkeiten zur Bearbeitung von Wundauflagen, insbesondere zum Vorsehen von abgeflachten Rändern, sind eingeschränkt, wenn auf einer beispielsweise als Polymerschaumstofflage ausgebildeten porösen Lage zusätzlich eine oder mehrere Wundkontakt-Lagen, bspw. Hydrogel-Schichten, vorhanden sind. Dies kann bspw. zu Schwierigkeiten bei der Schaffung von abgeflachten Rändern führen.

Beim Einsatz thermischer Schneideverfahren kann das Hydrogel einen Teil der eingestrahlten Energie absorbieren und den Schneidevorgang behindern. Verfahren, die auf noch nicht vollständig ausgehärteten Polymer-Mischungen beruhen, können im Zusammenhang mit Gel-Schaumstoff-Laminaten überhaupt nicht eingesetzt werden.

Aufgabe der vorliegenden Erfindung ist es, ein Verfahren bereitzustellen zur Herstellung einer mehrlagigen Wundauflage mit dreidimensionalen Strukturen, insbesondere mit abgeflachten Rändern, bereitzustellen, wobei die Wundauflage insbesondere wenigstens eine poröse Lage und mindestens eine, vorzugsweise als Hydrogel-Schicht ausgebildete, Wundkontakt-Lage umfasst.

Diese Aufgabe wird erfindungsgemäß durch ein Verfahren zur Herstellung einer Wundauflage gemäß Anspruch 1 gelöst.

Das erfindungsgemäße Verfahren umfasst die folgenden Schritte, von denen vorzugsweise wenigstens zwei vorzugsweise mehrere, insbesondere alle, in der nachfolgend wiedergegebenen Reihenfolge durchgeführt werden:
Schritt i) Bereitstellen eines für medizinische Zwecke geeigneten Laminats mit einer ersten Außenseite und einer zweiten Außenseite, wobei das Laminat mindestens eine poröse Lage, vorzugsweise die ein Textil, insbesondere ein Fasergelege (Nonwoven) und/oder ein Fasergewebe(Woven), und/oder einen Polymerschaumstoff umfasst, sowie eine Wundkontakt-Lage, die sich von der porösen Lage unterscheidet, aufweist.
Schritt ii) Auflegen des Laminats auf eine Arbeitsfläche, wobei die erste Außenseite die Arbeitsfläche nach dem Auflegen flächig kontaktiert. Das Auflegen des Laminates im vorliegenden Sinne ist dabei als flächiges in Kontakt bringen zu verstehen.
Schritt iii) Aufbringen einer Schablone, die eine Kontaktseite und eine Oberseite aufweist, vorzugsweise wobei die Schablone mindestens einen Durchbruch aufweist, wobei die Kontaktseite der Schablone beim Aufbringen auf die der Arbeitsfläche abgewandte zweite Außenseite des Laminats aufgebracht wird. Die Schablone kann einstückig ausgeführt sein. Der Durchbruch kann bspw. als Loch in der Schablone ausgeführt sein. Im Sinne der Erfindung ist auch die Verwendung von mehrteiligen Schablonen. Die Schablone kann bspw. mehrere Schablonenabschnitte bzw. Schablonenteile umfassen. Ein Durchbruch kann bspw. ein Loch in einem Schablonenteil sein oder ein Durchbruch kann durch entsprechendes Auflegen der Schablonenabschnitte bzw. Schablonenteile zwischen den Schablonenteilen gebildet sein. Die Schablone kann auch als umlaufendes Band, das gegen das Laminat geführt wird ausgebildet sein.
Schritt iv) Anpressen der Schablone in Richtung der Arbeitsfläche, wobei das Laminat durch das Anpressen der Schablone in seiner Position auf der Arbeitsfläche gehalten, vorzugsweise und in seiner Erstreckung von der ersten Außenseite zur zweiten Außenseite komprimiert, wird. Durch das Anpressen der Schablone wird das Laminat gewissermaßen zwischen der Schablone und der Arbeitsfläche verklemmt. Die Schablone kann dabei ortsfest sein und die Arbeitsfläche mit darauf angeordnetem Laminat kann gegen die Schablone gepresst werden. Die Schablone kann jedoch auch mit Druck oder Zug gegen die ortsfeste Arbeitsfläche mit darauf angeordnetem Laminat gepresst werden.
Schritt v) Abtrennen eines Teils des Laminates, der über die Oberseite der Schablone hinausragt, vorzugsweise wobei das Abtrennen in einer zu der Arbeitsfläche parallelen Trennebene, vorzugsweise, die höchstens 5mm, insbesondere höchstens 3mm, insbesondere höchstens 2mm, zur Oberseite der Schablone beabstandet ist, erfolgt. Bspw. kann der Teil des Laminates, der abgetrennt wird an den Seiten der Schablone über deren Oberseite hinausstehen. Der Teil des Laminates, der abgetrennt wird, kann alternativ oder zusätzlich auch durch einen Durchbruch der Schablone über deren Oberseite hinausstehen.
Vorzugsweise Schritt vi) Zuschneiden der Ränder des Laminats und/oder Aufteilen des Laminats, insbesondere durch einen im Wesentlichen vertikal zu der Erstreckungsebene des Laminats geführten Schnitt. Insbesondere kann das Laminat in diesem Schritt in einzelne Wundauflagen aufgeteilt werden. Das Laminat kann aber auch eine einzelne Wundauflage sein.
Vorzugsweise Schritt vii) Beaufschlagen und/oder Imprägnieren des Laminats mit einer, vorzugsweise wundheilungsfördernden, Substanz oder Zusammensetzung.
Vorzugsweise Schritt viii) Aufbringen weiterer Lagen auf die erste und/oder die zweite Außenseite des Laminats.
Vorzugsweise Schritt ix) Sterilisieren des Laminats.
Vorzugsweise Schritt x) Verpacken der Wundauflage.

Hierdurch ist eine effiziente, genaue und zuverlässige Formgebung des Laminats und damit der späteren Wundauflage möglich. Insbesondere eignet sich das vorliegende Verfahren zur automatisierten Serienfertigung von Wundauflagen.

Die Wundkontakt-Lage kann die erste Außenseite des Laminats bilden. Denkbar ist auch, dass ein ablösbarer Liner, der vorzugsweise auf der Wundkontakt-Lage angeordnet ist, die erste Außenseite des Laminats bzw. der Wundauflage bildet. Ein derartiger Liner kann jedoch auch bspw. in Schritt viii) auf das Laminat, insbesondere die erste Außenseite, aufgebracht werden.

Die poröse Lage ist in der Wundauflage, die aus dem Laminat hergestellt wird, insbesondere zur Aufnahme und Speicherung von Flüssigkeit, insbesondere Wundsekreten, vorgesehen. Die poröse Lage ist dabei vorzugsweise derart ausgebildet, dass ein Flüssigkeitstransport und eine Speicherung von Flüssigkeit, insbesondere von Wundsekreten, durch Kapillarkräfte erfolgt.

Die Wundkontakt-Lage, die insbesondere vergleichsweise dünn gegenüber der porösen Lage ausgebildet ist (bspw. eine Dicke von höchstens einem Drittel, insbesondere einem Viertel, insbesondere einem Fünftel, der porösen Lage aufweist), ist bei der Wundauflage, die aus dem Laminat hergestellt wird, zum Kontaktieren der Wundoberfläche vorgesehen.

Typischerweise bildet die poröse Lage volumenbezogen den Hauptanteil des Laminats.

Die Schablone ist vorzugsweise plattenförmig ausgebildet. In einer Ausführungsform umfasst die Schablone einen Kunststoff bzw. besteht aus dem Kunststoff. Vorzugsweise umfasst die Schablone ein ferromagnetisches Metall bzw. besteht die Schablone aus dem ferromagnetischen Metall, so dass sie ferromagnetische Eigenschaften aufweist.

Beim Anpressen der Schablone gemäß Schritt iv) kann das Laminat in Richtung zur Arbeitsfläche hin komprimiert werden, vorzugsweise wird hierbei die poröse Lage komprimiert. Bevorzugt ist, wenn die Anpresskraft der Schablone derart auf die Eigenschaften des Laminats abgestimmt ist, dass die poröse Lage komprimiert wird und die Wundkontakt-Lage beim Anpressen nicht komprimiert wird.

Entsprechend der Form der Schablone werden Teile des Laminats zusammengedrückt, während andere Anteile nicht komprimiert werden. Wird beispielsweise eine Schablone eingesetzt, welche eine kleinere Fläche als das Laminat aufweist, so dass nicht von der Schablone bedeckte Randbereiche des Laminats vorhanden sind, so werden diese Randbereiche nicht komprimiert, während die zentralen Bereiche des Laminats zusammengedrückt werden.

Wird gemäß einem weiteren Beispiel eine Schablone mit einem Durchbruch bspw. einem in der Mitte der Fläche offenen und hinreichend großem Bereich eingesetzt, so erfolgt in diesem Bereich, also im Bereich des Durchbruchs, keine Kompression des Laminats, so dass das Laminat durch den Durchbruch in der Schablone hindurch über die Oberseite der Schablone hinausragen kann. Der Durchbruch kann auch zum äußeren Rand der Schablone hin offen sein. Vorzugsweise ist wenigstens ein Durchbruch vorgesehen, der bezüglich dem äußeren Rand der Schablone geschlossen ausgeführt ist.

Im Schritt v) befindet sich die Trennebene vorzugsweise unmittelbar über der Schablone. Das Abtrennen unmittelbar über der Schablone bewirkt, dass die von der Schablone bedeckten Anteile des Laminats nicht verändert werden, während die nicht von der Schablone bedeckten Anteile des Laminats in ihrer jeweiligen Dicke zugeschnitten werden. Wird, wie in dem obigen Beispiel bereits beschrieben, eine Schablone eingesetzt, welche die Randbereiche des Laminats nicht bedeckt, so ergeben sich durch das Schneiden abgeflachte Ränder. Wird eine Schablone eingesetzt, welche einen Durchbruch umfasst, so kann ein Laminat erhalten werden, welches auf seiner zweiten Außenseite eine Vertiefung bspw. eine Mulde aufweist. Die genannten Beispiele können auch kombiniert werden, so dass ein Laminat mit abgeflachten Rändern und einer, bspw. zentral angeordneten, Mulde erhältlich ist. Die Schablone im Sinne der Erfindung kann auch mehrere Durchbrüche umfassen, sodass, vorteilhafter Weise mehrere Wundauflagen in einem Schritt v) auf ihrer zweiten Außenseite bearbeitet werden können. Mehrere Durchbrüche in der Schablone können jedoch auch zur Herstellung von mehreren Vertiefungen auf der zweiten Außenseite einer einzelnen Wundauflage verwendet werden. Erhebungen auf der Wundauflage bzw. dem Laminat können bspw. geschaffen werden, indem lediglich kleine Bereiche des Laminats mit einer Schablone bedeckt werden und der überwiegende Teil des Laminats durch Abtrennen in seiner Dicke reduziert wird.

Im optionalen Schritt vi) kann der Schnitt vorzugsweise im Wesentlichen vertikal zu der Erstreckungsebene bzw. zur ersten Außenseite des Laminats geführt werden. Hierdurch kann das Laminat auf die gewünschte Größe der Wundauflage zugeschnitten und/oder die Ränder einem Feinschnitt unterzogen werden. Schritt vi) ermöglicht es auch, aus einer größeren Laminatbahn eine Vielzahl von Zuschnitten zu erhalten, welche dann jeweils eine individuelle Wundauflage bilden können. Der Schritt vi) kann auch mehrmals durchgeführt werden, bspw. um in einer bzw. zwei Durchführungen (bspw. in Längs und Querrichtung) eine Vielzahl von Zuschnitten zu erhalten, die bspw. in einer weiteren Durchführung des Schritts vi) auf eine genaue Größe, die gewünschte Größe der Wundauflage, zugeschnitten zu werden können.

Vorzugsweise wird die Wundkontakt-Lage bei Durchführung des Schritts vi) durchtrennt. Vorzugsweise wird die Wundkontakt-Lage bei Durchführung des Schritts v) nicht durchtrennt.

Grundsätzlich kann das erfindungsgemäße Verfahren manuell durchgeführt werden oder automatisiert. Vorzugsweise erfolgt eine automatisierte Durchführung des Verfahrens auf einer Fertigungsmaschine.

Teil der vorliegenden Erfindung, die jedoch nicht beansprucht wird, ist daher auch eine Fertigungsmaschine, die eingerichtet und ausgebildet ist, um das erfindungsgemäße Verfahren durchzuführen.

Im Sinne der vorliegenden Erfindung ist es bevorzugt, wenn die Wundauflage, vorzugsweise neben einer guten Haut- und Gewebeverträglichkeit, ein die Wundheilung förderndes feuchtes Milieu gewährleisten kann, wobei vorzugsweise überschüssige Flüssigkeit absorbiert werden soll. Weiterhin ist es vorteilhaft, wenn die Wundauflage über atraumatische Eigenschaften verfügt, damit ist gemeint, dass die Wundauflage sich beim Verbandswechsel entfernen lässt, ohne frisch entstandenes Wundgewebe zu beschädigen.

Die Wundkontakt-Lage kann hinsichtlich der erwünschten atraumatischen Eigenschaften angepasst sein als auch für die Bereitstellung eines die Wundheilung fördernden Milieus.

Vorzugsweise ist die Wundauflage, insbesondere die Wundkontakt-Lage, derart ausgebildet, dass ein alkalischer pH-Wert, insbesondere ein pH-Wert über 8, bei der Wundbehandlung vermieden wird. Vorteilhaft ist, wenn die Wundauflage ausgebildet ist, um überschüssige Flüssigkeit aus dem Wundbereich abzuleiten und vorzugsweise zu absorbieren, da überschüssige Flüssigkeit bzw. Wundexsudat zur Mazeration des Wundrandes führen kann, wenn sie nicht abgeführt wird.

Zum Abtrennen gemäß Schritt v) und/oder zum Zuschneiden und/oder Aufteilen gemäß Schritt vi) kann Ultraschall-Schneiden eingesetzt werden. Insbesondere zum Erzeugen abgeflachter Ränder der Wundauflage bzw. des Laminats eignet sich die Verwendung von Ultraschall-Schneiden.

Denkbar ist auch, dass die poröse Schicht mittels thermischen Schneidens bearbeitet wird.

Denkbar ist auch die Bearbeitung des Laminats, insbesondere der porösen Schicht mittels Wasserstrahl-Schneiden.

Vorzugsweise erfolgt bei dem erfindungsgemäßen Verfahren das Anpressen der Schablone gemäß Schritt iv) wenigstens teilweise, vorzugsweise vollständig, durch eine magnetische Kraft. Vorzugsweise umfasst die Schablone ein ferromagnetisches Material und/oder es wird vor der Durchführung von Schritt iv) ein Anpresselement, das ein ferromagnetisches Material umfasst, auf der Oberseite der Schablone angeordnet. Hierdurch kann eine konstruktiv einfach herstellbare Schablone verwendet werden und die Anpresskraft kann kontaktlos auf die Schablone übertragen werden. Dies macht das Verfahren flexibel und reduziert die Anzahl an fehleranfälligen Teilen bei der Durchführung des Verfahrens.

Denkbar ist die Verwendung einer mehrschichtig aufgebauten Schablone, die eine Lage aus einem ferromagnetisches Material umfasst.

Vorzugweise ist/sind in oder unter der Arbeitsfläche ein Permanentmagnet und/oder ein Elektromagnet angeordnet, so dass eine zur Arbeitsfläche hin orientierte magnetische Kraft, vorzugsweise auf die Schablone, ausgeübt werden kann. Über den Permanentmagneten bzw. den Elektromagneten kann die Schablone und/oder ein entsprechendes Anpresselement mit Magnetkraft beaufschlagt werden. Mit anderen Worten, die Schablone ist über Magnetkräfte in Richtung der Arbeitsfläche drängbar. Beispielsweise kann zunächst die Schablone auf das auf der Arbeitsfläche aufliegende Laminat aufgelegt werden und dann die Arbeitsfläche über den Permanentmagneten bzw. den Elektromagneten bewegt werden, wodurch die Schablone über die Magnetkräfte zur Arbeitsfläche hin bewegt wird. Denkbar ist auch ein Aufbringen der Schablone und ein zeitlich nachfolgendes Bestromen des unter der Schablone, bspw. in oder unter der Arbeitsfläche, angeordneten Elektromagneten.

Denkbar ist auch, dass die Schablone einen Magneten einer ersten Polung umfasst oder ein solcher auf ihr angeordnet ist und auf Seiten der Arbeitsfläche ein weiterer Magnet (bspw. ein Permanent oder ein Elektromagnet) mit einer zweiten Polung derart angeordnet ist, dass die eben genannten beiden Magneten sich anziehen, wodurch die Schablone zur Arbeitsfläche hin gedrängt wird.

Denkbar ist auch, dass die Schablone einen Magneten einer ersten Polung umfasst oder ein solcher mit ihr verbunden ist und auf der der Arbeitsfläche abgewandten Seite der Schablone ein weiterer Magnet (bspw. ein Permanent oder ein Elektromagnet) mit einer zweiten Polung derart angeordnet ist, dass die eben genannten beiden Magneten sich abstoßen, wodurch die Schablone zur Arbeitsfläche hin gedrängt wird.

In den eben beschriebenen Ausführungsformen mit Magneten, die Schablonenseitig angeordnet sind, sind die zweiten Magneten vorzugsweise schaltbar, bspw. als Elektromagneten, und/oder nur in einem Teilbereich einer Vorrichtung zur Durchführung des Verfahrens angeordnet, bspw. in einem Teilbereich eines Transportbands.

In einer Ausführungsform des erfindungsgemäßen Verfahrens ist die Arbeitsfläche auf einem, vorzugsweise umlaufenden, Transportband angeordnet. Vorzugsweise ist die Arbeitsfläche durch eine Oberfläche des umlaufenden Transportbands gebildet. Hierdurch können Wundauflagen besonders effizient und schnell mittels des Verfahrens hergestellt werden.

Vorzugsweise erfolgt das Abtrennen gemäß Schritt v), indem das in seiner Position auf der Arbeitsfläche gehaltene Laminat, entlang einer Transportrichtung gegen eine, vorzugsweise quer zur Transportrichtung erstreckte, feststehende Trenneinrichtung geführt wird. Vorzugsweise erfolgt die Bewegung des Laminats dabei mittels des Transportbandes.

Die feststehende Trenneinrichtung ist vorzugsweise als feststehende Klinge, als Bandmesser oder als rotierendes Messer ausgeführt. Dabei ist mit feststehender Trenneinrichtung gemeint, dass die Trenneinrichtung ortsfest angeordnet ist, und sich das Laminat gegenüber der Trenneinrichtung bewegt.

Denkbar ist auch, dass das Laminat mit der auf dem Laminat angeordneten Schablone ortsfest ist und eine bewegliche Trenneinrichtung über die Schablone geführt wird. Eine derartige Trenneinrichtung ist vorzugsweise als feststehende Klinge, als Bandmesser oder als rotierendes Messer ausgeführt.

Vorzugsweise weist die Arbeitsfläche wenigstens eine, vorzugsweise mehrere, Erhöhung(en) und/oder Vertiefung(en) auf. Insbesondere durch Erhöhungen in der Arbeitsfläche können mit geringem Druck Teile des Laminats durch etwaige Durchbrüche in der Schablone gedrückt werden. Vorzugsweise ist die Erhöhung bzw. sind die Erhöhungen in der Arbeitsfläche dabei derart angeordnet, dass sie je unter einem Durchbruch in der Schablone liegen, wenn die Schablone gemäß Schritt iii) auf das Laminat aufgelegt wird.

Vorzugsweise umfasst die poröse Lage einen, insbesondere offenzelligen, Polymerschaumstoff. Der Polymerschaumstoff kann ein retikulierter Polymerschaumstoff sein. Der Polymerschaumstoff wiederum kann ein Polyether, ein Polyester, ein Polyurethan, ein Polyurethan-Polyether-Copolymer, ein Polyvinylacetat, ein Polyvinylalkohol, ein Kollagen, ein Chitosan, ein Polyolefin, ein Silikon oder eine Mischung aus wenigstens zwei dieser Bestandteile umfasst.

In einer Ausführungsform umfasst der Polymerschaumstoff einen offenzelligen Polyurethanschaumstoff, der erhältlich ist durch Umsetzung einer Mischung umfassend die Komponenten (i) Polyisocyanat, (ii) Polyol, bevorzugt Polyesterpolyol, (iii) Treibmittel und (iv) Katalysator.

Als Polymerschaumstoffe werden üblicherweise Werkstoffe mit über die gesamte Masse verteilten Zellen (offen, geschlossen oder beides) verstanden. Somit weisen solche Werkstoffe üblicherweise eine Rohdichte (gemäß DIN EN ISO 845) auf, die niedriger ist als die Dichte der Gerüstsubstanz.

Eine Zelle ist der bei der Herstellung von Polymerschaumstoffen gebildete einzelne Hohlraum, der von Zellwänden und/oder Zellstegen teilweise oder vollständig umschlossen ist.

Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht.

Im Rahmen dieser Anmeldung bedeutet der Begriff offenzellig, dass im Polymerschaumstoff mindestens 60 % offene Zellen, bevorzugt mindestens 90 % offene Zellen, bevorzugt mindestens 98 % offene Zellen, insbesondere im Wesentlichen 100 % offene Zellen, bezogen auf die Gesamtzahl an Zellen, vorhanden sind.

Die Offenzelligkeit des Polymerschaumstoffs wird üblicherweise nach ASTM D 2856-87, Verfahren B) bestimmt. Unter Zellwand oder Zellmembran wird üblicherweise die die Zelle umschließende Wand verstanden. Als Zellsteg wird üblicherweise der Bereich der Zellwand verstanden, der mehr als zwei Zellen voneinander trennt. Bei dem offenzelligen Polymerschaumstoff kann es sich um einen retikulierten oder um einen nicht-retikulierten Polymerschaumstoff handeln. Unter einem retikulierten Polymerschaumstoff wird ein Polymerschaumstoff verstanden, der im Wesentlichen nur Zellstege aufweist. Bei einem retikulierten Polymerschaumstoff sind die Zellwände somit im Wesentlichen entfernt.

Der Polymerschaumstoff im Sinne der vorliegenden Erfindung kann beispielsweise eine Luftdurchlässigkeit von 1000 bis 8000 l/(m²sec), mehr bevorzugt von 1500 bis 6000 l/(m²sec), noch mehr bevorzugt von 2000 bis 5000 l/(m²sec), besonders bevorzugt von 2300 bis 4000 l/(m²sec) und insbesondere von 2400 bis 3300 l/(m²sec), gemessen gemäß DIN EN ISO 9237 (20 mm Prüfdicke, 20 cm² Prüffläche, 200 Pa Differenzdruck) aufweisen.

Die Rohdichte des Polymerschaumstoffs kann im Sinne der vorliegenden Erfindung beispielsweise zwischen 10 und 350 kg/m3, gemessen gemäß DIN EN ISO 845, betragen. Bevorzugt beträgt die Rohdichte 15 bis 65 kg/m3, insbesondere 20 bis 45 kg/m3.

Es ist auch denkbar, dass die poröse Lage zwei oder mehr Lagen aus einem oder mehreren der vorgenannten Polymerschaumstoffe umfasst. Die Lagen können übereinander und/oder nebeneinander, bspw. konzentrisch zueinander, angeordnet sein.

Der in der porösen Lage vorhandene Polymerschaumstoff kann mit einem Zusatz und/oder Hilfsstoff beschichtet oder imprägniert sein. Bei dem Zusatz oder der Imprägnierung kann es sich beispielsweise um eine die Wundheilung fördernde Salbengrundlage handeln, wobei die Salbengrundlage vorzugsweise zusätzlich einen Quellstoff umfasst. Exemplarisch wird auf die WO2012/167943 verwiesen, welche im Sinne der Erfindung geeignete Salbengrundlagen beschreibt und hiermit in die Offenbarung der vorliegenden Anmeldung bezüglich der Salbengrundlagen eingebunden wird.

Alternativ oder zusätzlich kann im Polymerschaumstoff eine antimikrobiell wirksame Substanz vorhanden sein, beispielsweise Polyhexanid. Bei der antimikrobiell wirksamen Substanz kann es sich auch um ein Antibiotikum handeln, beispielsweise um Oxytetracyclin oder um eine Kombination von Bacitracin und Neomycin.

Gegebenenfalls kann der Zusatz oder die Imprägnierung zusätzlich zum Antibiotikum oder alternativ eine entzündungshemmende Substanz umfassen, beispielsweise Hydrocortison. Gemäß einer Ausführungsform umfasst die antimikrobiell wirksame Substanz Silber oder eine Silber-Verbindung.

Die poröse Lage, insbesondere der Polymerschaumstoff, kann Silber, vorzugsweise in Form von Silberionen oder in Form von atomarem Silber, enthalten. Bevorzugt ist Silber in Form einer Silberbeschichtung auf der Oberfläche der porösen Lage aufgebracht. Alternativ kann das Silber innerhalb der porösen Lage verteilt sein.

Beispielsweise kann bei offenzelligen Polymerschaumstoffen Silber bereits in die härtbare Zusammensetzung des Polymers mit eingebracht werden. Bevorzugt enthält das im Polymerschaumstoff vorhandene Polymer 0,000001 bis 0,1 Gew. % Silber, mehr bevorzugt 0,0001 bis 0,01 Gew.-% Silber, bezogen auf das Gesamtgewicht des jeweiligen Polymerschaumstoffs. Besonders bevorzugt enthält der Polymerschaumstoff 0,0001 bis 0,01 Gew.-% Silber, bezogen auf das Gesamtgewicht des Polymerschaumstoffs.

Des Weiteren kann die poröse Lage einen, insbesondere Partikel-förmigen, Zusatz umfassen, wobei es sich bei dem, insbesondere Partikel-förmigen, Zusatz insbesondere um ein quellfähiges Polymer handeln kann. Gemäß einer Ausführungsform umfasst die poröse Lage ein superabsorbierendes Polymer (SAP), insbesondere Partikel aus einem superabsorbierenden Polymer.

Vorzugsweise weist die Wundkontakt-Lage eine Vielzahl von über die Fläche der Wundkontakt-Lage verteilten Öffnungen oder eine netzartige Struktur auf. Die Öffnungen erstrecken sich vorzugsweise von der ersten Außenseite zur porösen Lage hin. Hierdurch kann effizient Flüssigkeit durch die Wundkontakt-Lage hindurch zur porösen Lage geleitet werden und beispielsweise unerwünschtes Wundexudat abgeführt werden.

Vorzugsweise umfasst die Wundkontakt-Lage eine Salbe, ein Silikon, ein Hydrogel und/oder ein Hydrokolloid. Hierdurch sind die mit dem Verfahren hergestellten Wundauflagen besonders förderlich für die Wundheilung.

Vorzugsweise umfasst die Wundkontakt-Lage ein Hydrogel, welches wiederum Polyacrylat, Polyurethan und/oder ein Polyurethan-Polyharnstoff-Copolymer umfasst, was die mit dem Verfahren hergestellten Wundauflagen besonders förderlich für die Wundheilung macht.

Das Laminat umfasst vorzugsweise mindestens eine die poröse Lage kontaktierende Hydrogel-Schicht. Diese Hydrogel-Schicht kann die Wundkontakt-Lage sein. Die Wundkontakt-Lage kann eine Hydrogel-Schicht umfassen oder aus einer Hydrogel-Schicht bestehen.

Hydrogel, das im Laminat gemäß dem erfindungsgemäßen Verfahren verwendet wird (beispielsweise für die Wundkontakt-Lage), umfasst bzw. ist vorzugsweise eine wasserhaltige Hydrogelmatrix. Die Hydrogelmatrix kann vorzugsweise mindestens 10 Gew.-%, insbesondere mindestens 30 Gew.-%, insbesondere mindestens 40 Gew.-% und ganz besonders bevorzugt mindestens 50 Gew.-% Wasser umfassen, wobei die Hydrogelmatrix weiterhin bevorzugt höchstens 90 Gew.-% und weiterhin bevorzugt höchsten 80 Gew.-% Wasser umfasst. Somit kann mittels des erfindungsgemäßen Verfahrens eine Wundauflage, vorzugsweise mit abgeflachten Rändern, bereitgestellt werden, welche neben einer porösen Lage eine Wundkontakt-Lage aufweist, die eine für natürliche Wundheilung ausreichende Menge Feuchtigkeit zur Verfügung stellt.

Als wasserhaltige Hydrogelmatrices können hierbei insbesondere Hydrogelmatrices verwendet werden, die eine zusammenhängende, diskrete Schicht bilden und unter Druck kein Wasser abgeben. Insbesondere sind im Zusammenhang mit der vorliegenden Erfindung Hydrogelmatrices geeignet, die ein Polyurethan-Polyharnstoff-Copolymer umfassen. Dieses Polyurethan-Polyharnstoff-Copolymer kann dabei insbesondere aus einem Präpolymer mit aliphatischen Diisocyanat-Gruppen und einem Polyamin auf Polyethylenoxidbasis gebildet werden. Insbesondere kann das Polyurethan-Polyharnstoff-Copolymer dabei aus einem Präpolymer mit Isophorondiisocyanat-Enden, einem Polyamin auf Polyethylenoxidbasis und Wasser gebildet werden. Diese Hydrogelmatrices sind besonders gut geeignet Wasser einzulagern und dieses Wasser an eine Wunde abzugeben. Weiterhin bevorzugt kann die wasserhaltige Hydrogelmatrix weiterhin mindestens einen mehrwertigen Alkohol aus der Gruppe der zweiwertigen, dreiwertigen, vierwertigen, fünfwertigen oder sechswertigen Alkohole umfassen. Insbesondere kann der Alkohol gewählt sein aus der Gruppe der Glykole, insbesondere Ethylenglykol oder Propylenglykol, sowie Sorbitol oder Glycerin oder Mischungen hiervon. Diese Alkohole sind als Feuchtigkeitsspender geeignet und stellen somit für die die Wunde umgebende Haut eine pflegende Komponente dar.

Dabei kann die wasserhaltige Hydrogelmatrix insbesondere 0 bis 50 Gew.-% eines mehrwertigen Alkohols umfassen. Insbesondere umfasst die Hydrogelmatrix 5 bis 40 Gew.-% eines mehrwertigen Alkohols und ganz besonders bevorzugt 10 bis 30 Gew.-% eines mehrwertigen Alkohols.

Darüber hinaus kann vorgesehen sein, dass die wasserhaltige Hydrogelmatrix insbesondere mindestens ein Salz umfasst. Insbesondere ist hierbei vorgesehen, dass die Hydrogelmatrix ein anorganisches Salz umfasst. Besonders geeignet sind in diesem Zusammenhang Chloride, Iodide, Sulfate, Hydrogensulfate, Karbonate, Hydrogenkarbonate, Phosphate, Dihydrogenphosphate oder Hydrogenphosphate der Alkali- und Erdalkalimetalle. Die Hydrogelmatrix kann Natriumchlorid, Kaliumchlorid, Magnesiumchlorid, Kalziumchlorid oder Mischungen hiervon umfassen. Diese Salze simulieren das Elektrolyt-Gemisch in einem von einer Wunde abgegebenen Wundserum. Damit stellt eine diese Salze umfassende Hydrogelmatrix einer Wunde ein wundheilungsförderndes Klima zur Verfügung.

Es kann vorgesehen sein, dass die Hydrogelmatrix 0 bis 5 Gew.-% eines oder mehrerer Salze(s) umfasst. Insbesondere umfasst die Hydrogelmatrix 0,1 bis 3 Gew.-% eines Salzes und ganz besonders bevorzugt 0,5 bis 1,5 Gew.-% eines Salzes.

Im Sinne der Erfindung ist, wenn das Laminat eine Hydrogelmatrix umfasst, deren Schichtdicke 0,1 bis 5,0 mm aufweist. Insbesondere weist damit eine erfindungsgemäße Wundauflage eine Wundkontakt-Lage mit einer Schichtdicke von 0,1 bis 5,0 mm, insbesondere von 0,5 bis 5,0 mm, vorzugsweise von 0,5 bis 3,0 mm auf. Wundauflagen mit Wundkontakt-Lagen mit solchen Schichtdicken zeigen einerseits keine Wundverklebung und andererseits die Fähigkeit ein von einer Wunde abgegebenes Wundexsudat aufzunehmen und an die absorbierende Schicht weiterzuleiten. Diese Schichtdicken können an jeder Stelle der Wundkontaktschicht gleich sein oder in verschiedenen Bereichen der Wundkontaktschicht verschiedene Werte annehmen.

Die Hydrogelmatrix kann Kanäle umfassen, insbesondere konische Kanäle. Die Kanäle können zum Durchtritt von Flüssigkeiten von der ersten Außenseite des Laminats, insbesondere der Wundkontakt-Lage, zur porösen Lage ausgebildet und angeordnet sein. Die Kanäle können insbesondere einen verbesserten Durchtritt für Wundexsudat bereitstellen.

Die Kanäle können einen elliptischen oder einen kreisförmigen Querschnitt aufweisen, d.h. dass die Kanäle eine kreisförmige oder elliptische Öffnung sowohl an der der ersten als auch an der zweiten Seite der Hydrogelmatrix aufweisen, wobei die kreisförmige oder elliptische Öffnung auf der ersten und der zweiten Seite verschieden groß sein können. Es kann jedoch auch vorgesehen sein, dass die Kanäle einen dreieckigen, rechteckigen, quadratischen, fünfeckigen, sechseckigen oder einen anderen vieleckigen Querschnitt aufweisen. Die erste Seite kann Öffnungen aufweisen, die im Vergleich zu der auf der zweiten Seite befindliche Öffnung größer ist.

Vorzugsweise wird nach dem Zuschneiden gemäß Schritt vi) des Laminats auf die zweite Außenseite eine Backing-Lage aufgebracht. Die Backing-Lage weist vorzugsweise eine klebende Beschichtung auf. Vorzugsweise überragt die Backing-Lage das Laminat wenigstens bereichsweise, vorzugsweise über seine gesamte flächige Erstreckung, derart, dass die hergestellte Wundauflage durch die eine klebende Beschichtung aufweisende Backing-Lage einen Kleberand aufweist. Vorzugsweise ist die Backing-Lage fluiddicht ausgeführt.

Vorzugsweise ist die erste Außenseite des Laminats bzw. der Wundauflage durch einen ablösbaren Liner gebildet.

Im Sinne der Erfindung ist auch, wenn ein ablösbarer Liner, vorzugsweise nach dem Zuschneiden und/oder Aufteilen gemäß Schritt vi) oder vor dem Auflegen des Laminats auf die Arbeitsfläche gemäß Schritt ii), auf die erste Außenseite, vorzugsweise auf die Wundkontakt-Lage, aufgebracht wird. Ein Aspekt der vorliegenden Erfindung ist auch eine Wundauflage, vorzugsweise mit abgeschrägten Kanten, wobei die Wundauflage, nach einem Verfahren gemäß einem oder mehreren der voranstehenden Ansprüche hergestellt ist, insbesondere wobei die Wundauflage auf der zweiten Außenseite Vertiefungen und/oder Erhebungen aufweist.

Die eben genannte Wundauflage weist wenigstens eine Vertiefung im mittleren Bereich der Wundauflage auf. Vorzugsweise sind in der Vertiefung im mittleren Bereich der Wundauflage ein Superabsorber und/oder ein pH-Indikator und/oder eine pharmazeutisch wirksame Substanz angeordnet. Weitere Merkmale, Anwendungsmöglichkeiten und Vorteile der Erfindung ergeben sich aus der nachfolgenden Beschreibung von Ausführungsbeispielen der Erfindung, die anhand der Zeichnung erläutert werden, wobei die Merkmale sowohl in Alleinstellung als auch in unterschiedlicher Kombination für die Erfindung wichtig sein können, ohne dass hierauf nochmals explizit hingewiesen wird. Es zeigen:
- Figur 1: einen Ausschnitt aus einem Laminat, das für medizinische Zwecke geeignet ist;
- Figur 2: eine schematische Darstellung der Durchführung von Schritten des Verfahrens;
- Figur 3: eine schematische Illustration einer Ausführungsform des erfindungsgemäßen Verfahrens;
- Figur 4: eine weitere schematische Illustration einer Ausführungsform des erfindungsgemäßen Verfahrens; und
- Figur 5: eine Illustration eines Verfahrensschritts vi).

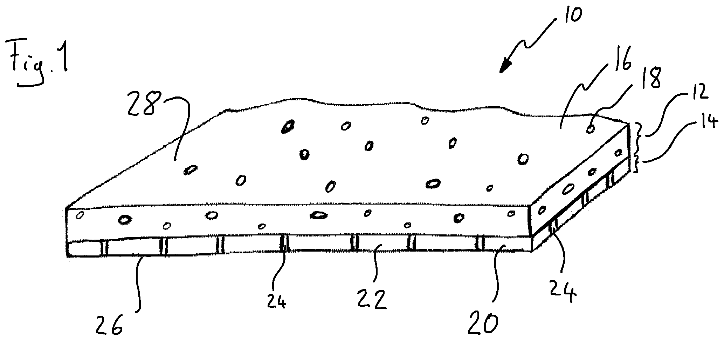

Figur 1 zeigt ein Laminat 10, das für medizinische Zwecke geeignet ist, wie es typischerweise zur Durchführung des erfindungsgemäßen Verfahrens Verwendung findet. Das Laminat 10 ist vorliegend nur bereichsweise dargestellt.

Das Laminat 10 umfasst in der in Figur 1 gezeigten Ausführungsform eine poröse Lage 12 und eine Wundkontaktlage 14. Die poröse Lage 12 ist vorliegend durch einen offenzelligen Polymerschaumstoff 16 gebildet. An den Oberflächen des Polymerschaumstoffs 16 sind Poren 18 symbolisch angedeutet.

Die Wundkontaktlage 14 ist vorliegend als Hydrogelschicht 20 ausgebildet. Andere Arten von Wundkontaktlagen sind ebenso denkbar im Sinne der vorliegenden Erfindung. Die Hydrogelschicht 20 ist vorliegend als wasserhaltige Hydrogelmatrix 22 ausgebildet. Die als Hydrogelschicht 20 ausgebildete Wundkontaktlage 14 steht in direktem Kontakt mit der porösen Lage 12.

Die Wundkontaktlage 14 weist eine Vielzahl von über die Fläche der Wundkontaktlage 14 verteilten Öffnungen 24 auf, die in Figur 1 symbolisch dargestellt sind. Anstatt oder zusätzlich zu den Öffnungen 24 kann die Wundkontaktlage 14 auch eine netzartige Struktur aufweisen.

Die Öffnungen 24 und/oder die netzartige Struktur sind vorteilhafterweise derart ausgebildet, dass Flüssigkeit von einer ersten Außenseite 26 des Laminats 10 durch die Wundkontaktlage 14 zur porösen Lage 12 über Kapilarkräfte transportiert werden kann.

Eine zweite Außenseite 28 des Laminats 10 wird im vorliegenden Beispiel durch die der Wundkontaktlage 14 abgewandten Seite der porösen Lage 12 gebildet.

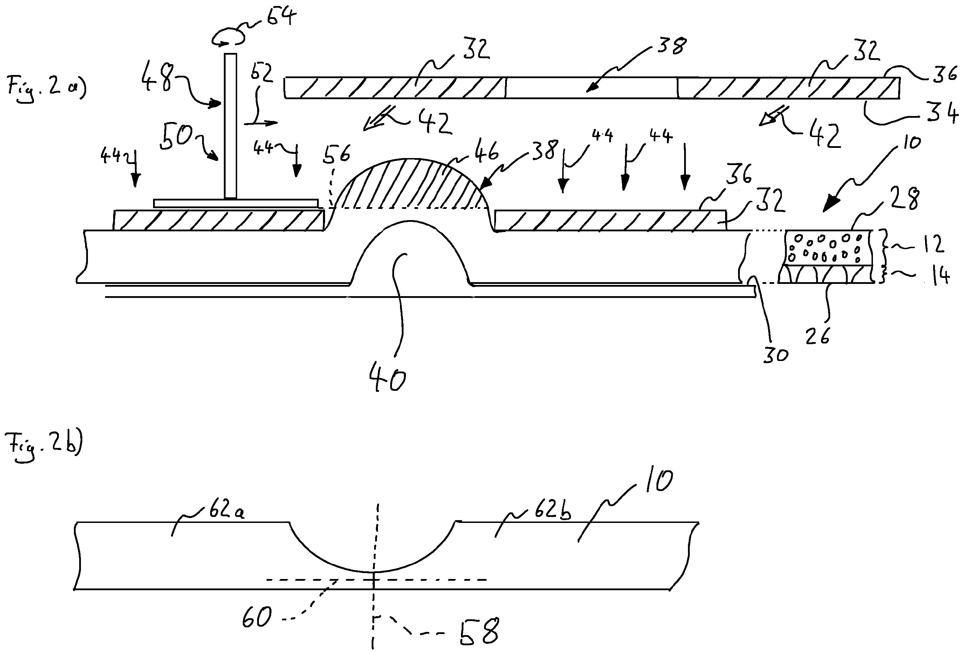

In Figur 2 ist die Durchführung des erfindungsgemäßen Verfahrens schematisch illustriert. In einem ersten Schritt i) wurde das Laminat 10 bereitgestellt.

In einem zweiten Schritt wurde das Laminat 10 mit seiner ersten Außenseite 26 auf eine Arbeitsfläche 30 aufgelegt. Die erste Außenseite 26 kontaktiert in diesem Zustand die Arbeitsfläche 30 flächig.

In einem dritten Schritt III) wurde eine Schablone 32, die eine Kontaktseite 34 sowie eine Oberseite 36 umfasst, aufgebracht. Die Schablone 32 weist einen Durchbruch 38 auf. Die Arbeitsfläche 30 weist eine Erhöhung 40 auf. Beim Platzieren der Schablone 32, welches in Figur 2a) über Pfeile mit dem Bezugszeichen 42 symbolisch dargestellt ist, wird der Durchbruch 38 über der Erhöhung 40 platziert. Im vorliegenden Beispiel drängt die Erhöhung 40 das Laminat 10 durch den Durchbruch 38 der Schablone 32 hindurch, so dass ein Teil 46 des Laminats 10 über die Oberseite 36 der Schablone 32 hinausragt.

In einem vierten Schritt iv) des Verfahrens wird die Schablone 32 in Richtung der Arbeitsfläche 30 angepresst, was durch Pfeile mit dem Bezugszeichen 44 symbolisch dargestellt ist.

Durch das Anpressen wird im Beispiel von Figur 2 das Laminat 10 in seiner Position auf der Arbeitsfläche 30 gehalten. Im Beispiel von Figur 2 wird das Laminat 10 dabei im Wesentlichen nicht komprimiert.

In einem fünften Schritt v) wird der Teil 46 des Laminats 10, der über die Oberseite 36 der Schablone 32 hinausragt, abgetrennt. Der abgetrennte bzw. abzutrennende Teil 46 ist in Figur 2a schraffiert dargestellt.

Das Abtrennen gemäß dem fünften Schritt V) erfolgt mittels einer Trenneinrichtung 48. In dem Beispiel von Figur 2a ist die Trenneinrichtung 48 als verfahrbares rotierendes Messer 50 ausgebildet. Die Verfahrbarkeit des Messers 50 ist durch einen Pfeil mit dem Bezugszeichen 52 dargestellt und die Rotation des Messers 50 durch einen Pfeil mit dem Bezugszeichen 54.

Das Abtrennen des Teils 46 des Laminats 10 erfolgt in einer Trennebene 56, die in Figur 2a durch eine unterbrochene Linie symbolisch dargestellt ist. Die Trennebene 56 ist vorliegend im Wesentlichen parallel zur Erstreckung der Arbeitsfläche 30 sowie zur Erstreckung der Schablone 32 angeordnet. Die Trennebene 56 ist im vorliegenden Beispiel weniger als 2 mm zur Oberseite 36 der Schablone 32 beabstandet. Im Sinne der Erfindung ist jedoch auch eine größere Beabstandung der Trennebene 56 zur Oberseite 36 der Schablone 32 möglich.

In einem sechsten Schritt VI) wird das Laminat 10 entlang einer Aufteillinie 58 aufgeteilt. Die Aufteillinie 58 erstreckt sich im Wesentlichen vertikal zur Erstreckungsebene 60 des Laminats 10. Durch die Aufteilung entlang der Aufteillinie 58 wird das Laminat 10 vorliegend in mehrere Wundauflagen 62 aufgeteilt, von denen in Figur 2b eine erste die teilweise dargestellte Wundauflage 62a und eine zweite die teilweise dargestellte Wundauflage 62b ist.

Bspw. im Anschluss an das Zuschneiden bzw. bereits im Anschluss an den Schritt V) kann das Laminat 10 bzw. die Wundauflagen 62 mit einer wundheilungsfördernden Substanz oder Zusammensetzung beaufschlagt und/oder imprägniert werden. Möglich ist auch, dass weitere Lagen auf die erste und/oder die zweite Außenseite 34, 36 des Laminats 10 aufgebracht werden. Dies kann in Schritten vii) und viii) erfolgen, welche vorzugsweise nach der Durchführung des Schritts vi) durchgeführt werden. In einem Schritt ix) kann das Laminat 10 bzw. die Wundauflagen 62 sterilisiert werden.

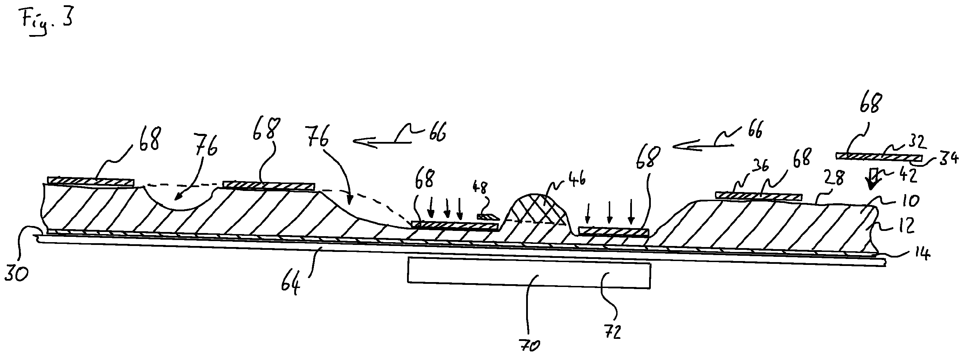

In Figur 3 ist gezeigt wie zunächst die Schablone 32 auf das Laminat 10 aufgebracht wird, was durch den Pfeil mit dem Bezugszeichen 42 schematisch angedeutet ist. Im Ausführungsbeispiel von Figur 3 ist die Arbeitsfläche 30 als Oberseite eines Transportbands 64 ausgeführt.

Das Transportband 64 bewegt das Laminat 10 entlang einer Transportrichtung 66. Die Transportrichtung 66 ist in Figur 3 durch Pfeile mit entsprechendem Bezugszeichen symbolisch dargestellt. Die Schablone 32 ist im Ausführungsbeispiel von Figur 3 durch mehrere miteinander unverbundene Schablonenabschnitte 68 gebildet.

Die einzelnen Schablonenabschnitte 68 werden in einer gewünschten Anordnung zueinander auf das Laminat 10 aufgebracht, so dass sie jeweils die zweite Außenseite 28 des Laminats 10 mit ihrer Kontaktseite 34 kontaktieren. Nachdem die Schablonen 32 bzw. die einzelnen Schablonenabschnitte 68 auf das Laminat 10 aufgebracht sind, bewegt das Transportband 64 das Laminat 10 mit den darauf liegenden Schablonenabschnitten 68 entlang der Transportrichtung 66.

In Transportrichtung 66 auf den Aufbringort der Schablonenabschnitte 68 nachfolgend ist ein Magnet 70 unterhalb der Arbeitsfläche 30 angeordnet. Der Magnet 70 ist vorliegend als Elektromagnet 72 ausgebildet. Ist der Elektromagnet 72 bestromt, so zieht er die ein ferromagnetisches Material umfassende Schablonenabschnitte 68 magnetisch an die Arbeitsfläche 30 heran. Durch dieses Heranziehen wird die poröse Lage 12 des Laminats 10 in seiner Erstreckung von der Arbeitsfläche zur zweiten Außenseite 28 hin komprimiert. Die magnetische Kraft des Elektromagneten 72 ist dabei vorzugsweise wie in Figur 3 illustriert, derart eingestellt, dass die Wundkontaktlage 14 im Wesentlichen nicht komprimiert wird.

In den Zwischenräumen zwischen den einzelnen Schablonenabschnitten 68, die Durchbrüche 38 (siehe Figur 2a) bilden, wird die poröse Lage 12 weniger komprimiert als unterhalb der Schablonenabschnitte 68. Die über die Oberseite 36 der Schablonenabschnitte 68 herausragenden Teile 46 des Laminats 10 werden durch die Bewegung des Transportbandes 64 gegen eine Trenneinrichtung 48, die vorliegend als feststehende Klinge ausgeführt ist, geführt. Hierdurch werden die über die Oberseite 36 der Schablonenteile 68 überstehenden Teile des Laminats 10 abgetrennt. Wenn die Schablonenabschnitte 68 durch die Bewegung des Transportbandes 64 entlang der Transportrichtung 66 von dem Magneten 70 wegbewegt werden, so dehnt sich das Laminat 10 wieder in seine ursprüngliche Erstreckung aus und es ist ersichtlich, dass an den Stellen, an denen die Teile 46 des Laminats abgetrennt wurden, das Laminat 10 nun eine Ausnehmung bzw. Mulde 76 aufweist.

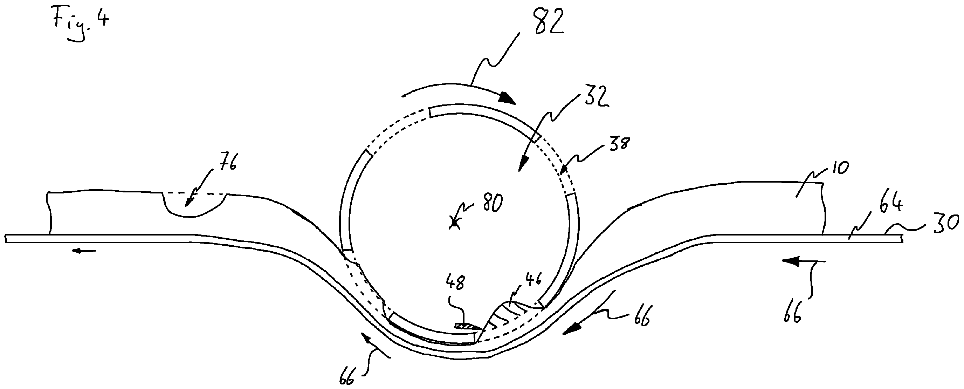

Figur 4 zeigt eine weitere Alternative zur Durchführung des erfindungsgemäßen Verfahrens. Bei der Version von Figur 4 ist das Laminat 10 auf ein flexibles Transportband 64 aufgelegt. Die Oberseite des Transportbands 64 bildet gleichzeitig die Arbeitsfläche 30. Im Beispiel von Figur 4 ist die Schablone 32 als Rolle ausgeführt, die sich um eine Drehachse 80 dreht. Die Schablone 32 hat mehrere Durchbrüche 38. Im Innern der Schablone 32, deren Rotation durch einen entsprechenden Pfeil 82 dargestellt ist, ist eine Trenneinrichtung 48 angeordnet, die als feststehende Klinge ausgeführt ist. Die Teile des Laminats 10 bzw. dessen poröser Lage 12, die unter den Durchbrüchen 38 angeordnet sind, erstrecken sich durch den Druck des elastischen Transportbands 64 durch die Durchbrüche 38 hindurch und werden durch die Trenneinrichtung 48 abgetrennt. Hierdurch erhält das Laminat 10 Mulden bzw. Ausnehmungen 76, wenn der jeweilige Abschnitt des Laminats die rotierende Schablone 32 passiert hat.

Im Beispiel von Figur 4 erfolgt das Anpressen der Schablone gemäß Schritt iv) durch die elastische Nachgiebigkeit des Transportbandes 64. Die Schablone 32 im Beispiel von Figur 4 ist ortsfest und unnachgiebig. Denkbar ist jedoch auch die Verwendung eines starren Transportbands 64 und einer nachgiebigen Schablone 32.

Allgemein ist mit dem Anpressen der Schablonen in Richtung der Arbeitsfläche gemäß Schritt iv) eine Druckbeaufschlagung des Laminats zwischen Schablone 32 und Arbeitsfläche 30 gemeint. Hierzu kann zum einen die Schablone 32 zur Arbeitsfläche hin bewegt werden und/oder die Arbeitsfläche 30 zur Schablone 32 hin bewegt werden. Bei dem eben genannten Anpressen wird vorzugsweise das Laminat 10, vorzugsweise ausschließlich dessen poröse Lage 12, komprimiert. Im Sinne der Erfindung ist jedoch mit einem Anpressen auch eine Druckbeaufschlagung des Laminats 10 durch die Schablone 32 gemeint, bei der das Laminat 10 nicht komprimiert wird, sondern lediglich in seiner Position auf der Arbeitsfläche 30 fixiert wird. Abzutrennende Teile 46 können in diesem Fall bspw. wie im Bsp. von Fig.2 gezeigt durch Erhöhungen in der Arbeitsfläche über die Oberseite 36 der Schablone 32 gedrängt werden.

Je nach Ausbildung und Anordnung der Durchbrüche 38 in der Schablone 32 oder einzelner Abschnitte 68 der Schablone 32 können komplexe dreidimensionale Strukturen in das Laminat 10 eingebracht werden.

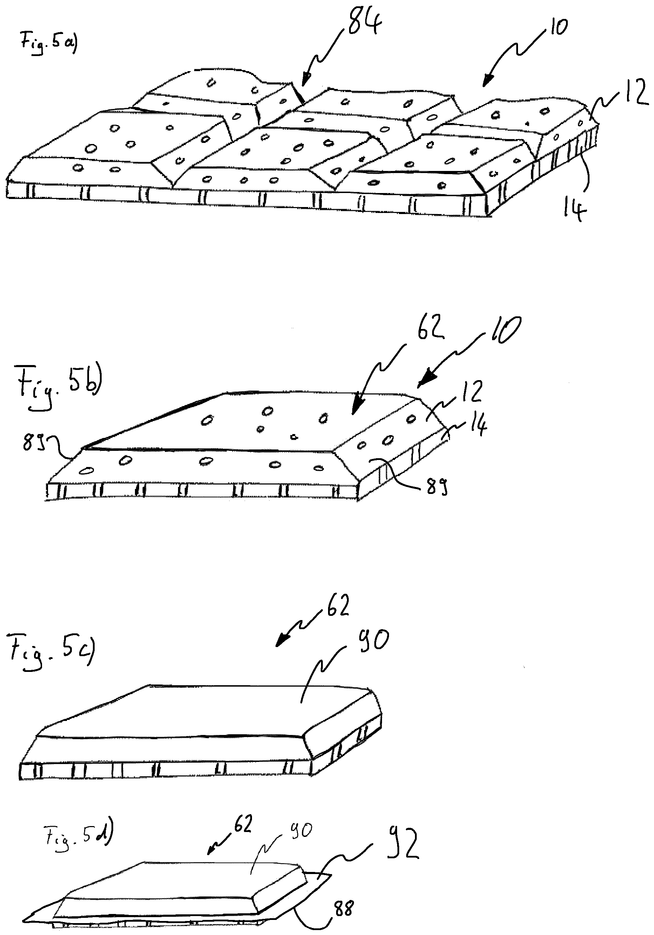

In Figur 5a ist eine beispielhafte Konturierung eines Laminats 10 gezeigt. Das Laminat 10 weist quer und längs verlaufende Vertiefungen 84 auf. Die Vertiefungen 84 erstrecken sich durch die poröse Lage 12 und sind in einem Schritt v) in die poröse Lage 12 des Laminats 10 eingebracht worden.

In einem Schritt vi) kann das Laminat 10 im Beispiel von Figur 5a entlang der Vertiefungen 84 vollständig durch einen im Wesentlichen vertikal zur Erstreckungsebene 60 des Laminats 10 verlaufenden Aufteilvorgang gemäß einem Schritt vi) in einzelne Wundauflagen 62 aufgeteilt werden.

Anders als in Figur 5b gezeigt, kann eine Wundauflage 62 jedoch auch über ihre flächige Erstreckung hinweg Vertiefungen 84 aufweisen. Die Wundauflage 62 weist abgeschrägte Kanten 89 bzw. Seitenflächen 89 auf. In Figur 5c ist gezeigt, dass auf die Wundauflage 62 aus Figur 5b eine Backing-Lage 90 aufgebracht werden kann. Die Backing-Lage 90 weist eine klebende Beschichtung 88 auf. Die Backing-Lage 90 ist im Beispiel von Figur 5c als Polyurethanfilm ausgebildet und auf der zweiten Außenseite 28 der Wundauflage 86 bzw. des Laminats 10 aufgebracht. Anders als im Beispiel von Figur 5c gezeigt, kann die Backing-Lage 90 das Laminat 10 auch wenigstens bereichsweise vorzugsweise über seine gesamte flächige Erstreckung derart überragen, dass die Wundauflage 62 einen Kleberand 92 aufweist. Eine Wundauflage 62 mit Backing-Lage 90, die einen Kleberand 92 bildet, ist in Figur 5d illustriert.

## Patentansprüche

1. Verfahren zur Herstellung einer Wundauflage (62), umfassend die Schritte
i) Bereitstellen eines für medizinische Zwecke geeigneten Laminats (10) mit einer ersten Außenseite (26) und einer zweiten Außenseite (28), wobei das Laminat (10) mindestens eine poröse Lage (12), vorzugsweise die ein Textil, insbesondere ein Fasergelege und/oder ein Fasergewebe, und/oder einen Polymerschaumstoff umfasst, sowie eine Wundkontakt-Lage (14), die sich von der porösen Lage (12) unterscheidet, aufweist,
ii) Auflegen des Laminats (10) auf eine Arbeitsfläche (30), wobei die erste Außenseite (26) die Arbeitsfläche (30) nach dem Auflegen flächig kontaktiert,
iii) Aufbringen einer Schablone (32), die eine Kontaktseite (34) und eine Oberseite (36) aufweist, vorzugsweise wobei die Schablone (32) mindestens einen Durchbruch (38) aufweist, wobei die Kontaktseite (34) der Schablone (32) beim Aufbringen auf die der Arbeitsfläche (30)abgewandte zweite Außenseite (28) des Laminats (10) aufgebracht wird,
iv) Anpressen der Schablone (32) in Richtung der Arbeitsfläche (30), wobei das Laminat (10) durch das Anpressen der Schablone (32) in seiner Position auf der Arbeitsfläche (30) gehalten, vorzugsweise und in seiner Erstreckung von der ersten Außenseite (26) zur zweiten Außenseite (28) komprimiert, wird,
v) Abtrennen eines Teils (46) des Laminates (10), der über die Oberseite (36) der Schablone (32) hinausragt, vorzugsweise wobei das Abtrennen in einer zu der Arbeitsfläche (30) parallelen Trennebene (56), vorzugsweise, die höchstens 5mm, insbesondere höchstens 3mm, insbesondere höchstens 2mm, zur Oberseite (36) der Schablone (32) beabstandet ist, erfolgt,
Vorzugsweise vi) Zuschneiden der Ränder des Laminats (10) und/oder Aufteilen des Laminats (10), um eine oder mehrere Wundauflagen (62) zu erhalten, wobei das Zuschneiden und/oder Aufteilen insbesondere durch einen im Wesentlichen vertikal zu der Erstreckungsebene (60) des Laminats (10) geführten Schnitt erfolgt,
Vorzugsweise vii) Beaufschlagen und/oder Imprägnieren des Laminats (10) bzw. der Wundauflage (62) mit einer, vorzugsweise wundheilungsfördernden, Substanz oder Zusammensetzung,
Vorzugsweise viii) Aufbringen weiterer Lagen auf die erste und/oder die zweite Außenseite des Laminats (10) bzw. der Wundauflage (62),
Vorzugsweise ix) Sterilisieren des Laminats (10) bzw. der Wundauflage (62) und
Vorzugsweise x) Verpacken der Wundauflage (62) bzw. der Wundauflagen (62),
vorzugsweise wobei die Schritte i) bis x), bzw. mehrere dieser Schritte in der eben genannten Reihenfolge erfolgen.

2. Verfahren nach Anspruch 1, wobei das Anpressen der Schablone (32) gemäß Schritt iv) wenigstens teilweise, vorzugsweise vollständig, durch eine magnetische Kraft erfolgt, vorzugsweise wobei die Schablone (32) ein ferromagnetisches Material umfasst oder vor der Durchführung von Schritt iv) ein Anpresselement, das ein ferromagnetisches Material umfasst, auf der Oberseite (36) der Schablone (32) angeordnet wird.

3. Verfahren nach einem der Ansprüche 1 oder 2, wobei in oder unter der Arbeitsfläche (30) ein Permanentmagnet und/oder ein Elektromagnet angeordnet ist bzw. sind, so dass eine zur Arbeitsfläche (30) hin orientierte magnetische Kraft, vorzugsweise auf die Schablone (32), ausgeübt werden kann.

4. Verfahren nach einem oder mehreren der voranstehenden Ansprüche, wobei die Arbeitsfläche (30) auf einem, vorzugsweise umlaufenden, Transportband (64) angeordnet ist, vorzugsweise durch eine Oberfläche des Transportbands (64) gebildet ist.

5. Verfahren nach einem oder mehreren der voranstehenden Ansprüche, wobei das Abtrennen gemäß Schritt v) erfolgt, indem das in seiner Position auf der Arbeitsfläche (30) gehaltene Laminat (10), vorzugsweise mittels des Transportbandes (64), entlang einer Transportrichtung (66) gegen eine, vorzugsweise quer zur Transportrichtung (66)erstreckte, feststehende Trenneinrichtung (48), vorzugsweise die als feststehende Klinge, als Bandmesser oder als rotierendes Messer ausgeführt ist, geführt wird.

6. Verfahren nach einem oder mehreren der voranstehenden Ansprüche, wobei die Arbeitsfläche (30) wenigstens eine, vorzugsweise mehrere, Erhöhung(en) (40) und/oder Vertiefung(en) aufweist.

7. Verfahren nach einem oder mehreren der voranstehenden Ansprüche, wobei die poröse Lage einen Polymerschaumstoff umfasst, welcher wiederum ein Polyether, ein Polyester, ein Polyurethan, ein Polyurethan-Polyether-Copolymer, ein Polyvinylacetat, ein Polyvinylalkohol, ein Kollagen, ein Chitosan, ein Polyolefin, ein Silikon oder eine Mischung aus wenigstens zwei dieser Bestandteile umfasst.

8. Verfahren nach einem oder mehreren der voranstehenden Ansprüche, wobei die Wundkontakt-Lage (14) eine Vielzahl von über die Fläche verteilten Öffnungen (24), vorzugsweise die sich von der ersten Außenseite (26) zur porösen Lage (12) hin erstrecken, oder eine netzartige Struktur aufweist.

9. Verfahren nach einem oder mehreren der voranstehenden Ansprüche, wobei die Wundkontakt-Lage (14) eine Salbe, ein Silikon, ein Hydrogel und/oder ein Hydrokolloid umfasst.

10. Verfahren nach einem oder mehreren der voranstehenden Ansprüche, wobei die Wundkontakt-Lage (14) ein Hydrogel umfasst, welches wiederum Polyacrylat, Polyurethan und/oder ein Polyurethan-Polyharnstoff-Copolymer umfasst.

11. Verfahren nach einem oder mehreren der voranstehenden Ansprüche, wobei nach dem Zuschneiden der Ränder des Laminats (10) und/oder dem Aufteilen des Laminats (10) gemäß Schritt vi) auf die zweite Außenseite (28) eine Backing-Lage (90) aufgebracht wird, vorzugsweise wobei die Backing-Lage (90) eine klebende Beschichtung (88) aufweist und die Backing-Lage (90) das Laminat (10) wenigstens bereichsweise, vorzugsweise über seine gesamte flächige Erstreckung, derart überragt, dass die Wundauflage (62) einen Kleberand (92) aufweist.

12. Verfahren nach einem oder mehreren der voranstehenden Ansprüche, wobei die erste Außenseite (26) des Laminats (10) bzw. der Wundauflage (62) durch einen ablösbaren Liner gebildet ist oder wobei ein ablösbarer Liner, vorzugsweise nach dem Zuschneiden der Ränder des Laminats (10) und/oder dem Aufteilen des Laminats (10) gemäß Schritt vi) oder vor dem Auflegen des Laminats (10) auf die Arbeitsfläche (30) gemäß Schritt ii), auf die ersten Außenseite (26), vorzugsweise auf die Wundkontakt-Lage (14), aufgebracht wird.

13. Wundauflage (62), vorzugsweise mit abgeschrägten Kanten (89), wobei die Wundauflage, nach einem Verfahren gemäß einem oder mehreren der voranstehenden Ansprüche hergestellt ist, insbesondere wobei die Wundauflage (62) auf der zweiten Außenseite (28) Vertiefungen (84) und/oder Erhebungen (40) aufweist,wobei die Wundauflage (62) wenigstens eine Vertiefung (84) im mittleren Bereich der Wundauflage (62) aufweist, vorzugsweise wobei in der Vertiefung im mittleren Bereich der Wundauflage (62) ein Superabsorber und/oder ein pH-Indikator und/oder eine pharmazeutisch wirksame Substanz angeordnet ist.

## Claims

1. Method for producing a wound dressing (62), comprising the steps of
i) providing a laminate (10) suitable for medical purposes having a first outer face (26) and a second outer face (28), wherein the laminate (10) has at least one porous layer (12), which preferably comprises a textile, in particular a laid fiber scrim and/or a woven fiber fabric, and/or a polymer foam, and has a wound contact layer (14) that is different from the porous layer (12),
ii) placing the laminate (10) onto a work surface (30), wherein the first outer face (26) is in contact with the work surface (30) in a planar manner after the placement,
iii) applying a template (32) having a contact face (34) and an upper face (36), preferably wherein the template (32) has at least one opening (38), wherein, during application, the contact face (34) of the template (32) is applied to the second outer face (28) of the laminate (10), which faces away from the work surface (30),
iv) pressing the template (32) toward the work surface (30), wherein the laminate (10) is held in its position on the work surface (30) by the pressing of the template (32), and preferably is compressed in its extension from the first outer face (26) to the second outer face (28),
v) separating a part (46) of the laminate (10) that projects beyond the upper face (36) of the template (32), preferably wherein the separation takes place in a separating plane (56) which is parallel to the work surface (30) and which is preferably spaced apart from the upper face (36) of the template (32) by at most 5 mm, in particular at most 3 mm, in particular at most 2 mm,
preferably vi) cutting the edges of the laminate (10) and/or dividing the laminate (10) in order to obtain one or more wound dressings (62), wherein the cutting and/or division takes place in particular by means of a cut guided substantially vertically to the extension plane (60) of the laminate (10),
preferably vii) applying and/or impregnating the laminate (10) or the wound dressing (62) with a substance or composition which preferably promotes wound healing,
preferably viii) applying further layers to the first and/or the second outer face of the laminate (10) or the wound dressing (62),
preferably ix) sterilizing the laminate (10) or the wound dressing (62) and
preferably x) packaging the wound dressing (62) or the wound dressings (62),
preferably wherein steps i) to x), or a plurality of these steps, take place in the sequence just mentioned.

2. Method according to claim 1, wherein the pressing of the template (32) according to step iv) takes place at least partially, preferably completely, by means of a magnetic force, preferably wherein the template (32) comprises a ferromagnetic material or a pressing element comprising a ferromagnetic material is arranged on the upper face (36) of the template (32) before step iv) is carried out.

3. Method according to either claim 1 or claim 2, wherein a permanent magnet and/or an electromagnet is/are arranged in or below the work surface (30), so that a magnetic force oriented toward the work surface (30) can be exerted, preferably on the template (32).

4. Method according to one or more of the preceding claims, wherein the work surface (30) is arranged on a preferably revolving conveyor belt (64) and is preferably formed by a surface of the conveyor belt (64).

5. Method according to one or more of the preceding claims, wherein the separation according to step v) is carried out by the laminate (10), which is held in its position on the work surface (30), preferably by means of the transport belt (64), being guided along a transport direction (66) toward a stationary separating device (48), which preferably extends transversely to the transport direction (66) and is preferably designed as a stationary blade, as a band knife or as a rotating blade.

6. Method according to one or more of the preceding claims, wherein the work surface (30) has at least one, preferably a plurality of, elevation(s) (40) and/or depression(s).

7. Method according to one or more of the preceding claims, wherein the porous layer comprises a polymer foam which in turn comprises a polyether, a polyester, a polyurethane, a polyurethane-polyether copolymer, a polyvinyl acetate, a polyvinyl alcohol, a collagen, a chitosan, a polyolefin, a silicone or a mixture of at least two of these constituents.

8. Method according to one or more of the preceding claims, wherein the wound contact layer (14) has a plurality of openings (24) distributed over the surface, which openings preferably extend from the first outer face (26) to the porous layer (12), or has a net-like structure.

9. Method according to one or more of the preceding claims, wherein the wound contact layer (14) comprises an ointment, a silicone, a hydrogel and/or a hydrocolloid.

10. Method according to one or more of the preceding claims, wherein the wound contact layer (14) comprises a hydrogel which in turn comprises polyacrylate, polyurethane and/or a polyurethane-polyurea copolymer.

11. Method according to one or more of the preceding claims, wherein, after the edges of the laminate (10) are cut and/or the laminate (10) is divided according to step vi), a backing layer (90) is applied to the second outer face (28), preferably wherein the backing layer (90) has an adhesive coating (88) and the backing layer (90) projects beyond the laminate (10) at least in regions, preferably over the entire planar extension thereof, such that the wound dressing (62) has an adhesive edge (92).

12. Method according to one or more of the preceding claims, wherein the first outer face (26) of the laminate (10) or the wound dressing (62) is formed by a detachable liner, or wherein, preferably after the edges of the laminate (10) are cut and/or the laminate (10) is divided according to step vi), or before the laminate (10) is placed on the work surface (30) according to step ii), a detachable liner is applied to the first outer face (26), preferably to the wound contact layer (14).

13. Wound dressing (62), preferably having beveled edges (89), wherein the wound dressing is produced according to a method according to one or more of the preceding claims, in particular wherein the wound dressing (62) has depressions (84) and/or elevations (40) on the second outer face (28), wherein the wound dressing (62) has at least one depression (84) in the central region of the wound dressing (62), preferably wherein a superabsorber and/or a pH indicator and/or a pharmaceutically active substance is arranged in the depression in the central region of the wound dressing (62).

## Revendications

1. Procédé pour la fabrication d'un pansement (62), comprenant les étapes
i) de fourniture d'un stratifié (10) adapté à des fins médicales avec une première face extérieure (26) et une deuxième face extérieure (28), dans lequel le stratifié (10) présente au moins une couche poreuse (12), de préférence qui comprend un textile, en particulier une nappe de fibres et/ou un tissu fibreux, et/ou une mousse polymère, ainsi qu'une couche en contact avec la plaie (14), qui se différencie de la couche poreuse (12),
ii) de pose du stratifié (10) sur une surface de travail (30), dans lequel la première face extérieure (26) est en contact plan avec la surface de travail (30) après la pose,
iii) d'application d'un gabarit (32), qui présente une face de contact (34) et une face supérieure (36), de préférence dans lequel le gabarit (32) présente au moins un passage (38), dans lequel la face de contact (34) du gabarit (32) lors de l'application est appliquée sur la deuxième face extérieure (28) du stratifié (10) opposée à la surface de travail (30),
iv) de pressage du gabarit (32) en direction de la surface de travail (30), dans lequel le stratifié (10) est maintenu par le pressage du gabarit (32) dans sa position sur la surface de travail (30), de préférence et comprimé dans son étendue de la première face extérieure (26) à la deuxième face extérieure (28),
v) de séparation d'une partie (46) du stratifié (10), qui fait saillie de la face supérieure (36) du gabarit (32), de préférence dans lequel la séparation s'effectue dans un plan de séparation (56) parallèle à la surface de travail (30), de préférence, qui est espacé au maximum de 5 mm, en particulier au maximum de 3 mm, en particulier au maximum de 2 mm, de la face supérieure (36) du gabarit (32),
de préférence vi) de découpe des bords du stratifié (10) et/ou partage du stratifié (10), afin d'obtenir un ou plusieurs pansements (62), dans lequel la découpe et/ou le partage s'effectue en particulier par une coupe guidée sensiblement verticalement par rapport au plan d'étendue (60) du stratifié (10),
de préférence vii) de sollicitation et/ou l'imprégnation du stratifié (10) ou du pansement (62) avec une substance ou composition, de préférence favorisant la cicatrisation,
de préférence viii) d'application d'autres couches sur la première et/ou la deuxième face extérieure du stratifié (10) ou du pansement (62),
de préférence ix) de stérilisation du stratifié (10) ou du pansement (62) et
de préférence x) d'emballage du pansement (62) ou des pansements (62),
de préférence dans lequel les étapes i) à x), ou plusieurs de ces étapes s'effectuent dans l'ordre venant d'être cité.

2. Procédé selon la revendication 1, dans lequel le pressage du gabarit (32) selon l'étape iv) s'effectue au moins en partie, de préférence entièrement, au moyen d'une force magnétique, de préférence dans lequel le gabarit (32) comprend un matériau ferromagnétique ou avant la réalisation de l'étape iv) un élément de pressage, qui comprend un matériau ferromagnétique, est disposé sur la face supérieure (36) du gabarit (32).

3. Procédé selon l'une quelconque des revendications 1 ou 2, dans lequel un aimant permanent et/ou un électroaimant est ou sont disposés dans ou sous la surface de travail (30), de sorte qu'une force magnétique orientée vers la surface de travail (30), peut être exercée de préférence sur le gabarit (32) .

4. Procédé selon l'une ou plusieurs des revendications précédentes, dans lequel la surface de travail (30) est disposée sur une bande de transport (64), de préférence rotative, de préférence est formée par une surface de la bande de transport (64).

5. Procédé selon l'une ou plusieurs des revendications précédentes, dans lequel la séparation selon l'étape v) s'effectue du fait que le stratifié (10) maintenu dans sa position sur la surface de travail (30) est guidé, de préférence au moyen de la bande de transport (64), le long d'une direction de transport (66) contre un dispositif de séparation (48) fixe, de préférence étendu transversalement par rapport à la direction de transport (66), de préférence qui est conçu sous la forme d'une lame fixe, sous la forme d'un couteau à bande ou sous la forme d'un couteau rotatif.

6. Procédé selon l'une ou plusieurs des revendications précédentes, dans lequel la surface de travail (30) présente au moins un(e), de préférence plusieurs, élévation(s) (40) et/ou creux.

7. Procédé selon l'une ou plusieurs des revendications précédentes, dans lequel la couche poreuse comprend une mousse polymère, laquelle comprend pour sa part un polyéther, un polyester, un polyuréthane, un copolymère polyuréthane-polyéther, un acétate de polyvinyle, un alcool polyvinylique, un collagène, un chitosan, une polyoléfine, un silicone ou un mélange d'au moins deux de ces composants.

8. Procédé selon l'une ou plusieurs des revendications précédentes, dans lequel la couche en contact avec la plaie (14) présente une pluralité d'ouvertures (24) réparties sur la surface, de préférence qui s'étendent de la première face extérieure (26) à la couche poreuse (12), ou une structure réticulée.

9. Procédé selon l'une ou plusieurs des revendications précédentes, dans lequel la couche en contact avec la plaie (14) comprend un onguent, un silicone, un hydrogel et/ou un hydrocolloïde.

10. Procédé selon l'une ou plusieurs des revendications précédentes, dans lequel la couche en contact avec la plaie (14) comprend un hydrogel, lequel comprend pour sa part du polyacrylate, polyuréthane et/ou un copolymère polyuréthane-polyurée.

11. Procédé selon l'une ou plusieurs des revendications précédentes, dans lequel après la découpe des bords du stratifié (10) et/ou le partage du stratifié (10) selon l'étape vi) une couche de support (90) est appliquée sur la deuxième face extérieure (28), de préférence dans lequel la couche de support (90) présente un revêtement adhésif (88) et la couche de support (90) fait saillie du stratifié (10) au moins par endroits, de préférence sur toute son étendue plane, de telle sorte que le pansement (62) présente un bord adhésif (92) .

12. Procédé selon l'une ou plusieurs des revendications précédentes, dans lequel la première face extérieure (26) du stratifié (10) ou du pansement (62) est formée par un liner détachable ou dans lequel un liner détachable, de préférence après la découpe des bords du stratifié (10) et/ou le partage du stratifié (10) selon l'étape vi) ou avant la pose du stratifié (10) sur la surface de travail (30) selon l'étape ii), est appliqué sur la première face extérieure (26), de préférence sur la couche en contact avec la plaie (14).

13. Pansement (62), de préférence avec des arêtes biseautées (89), dans lequel le pansement est fabriqué selon un procédé selon l'une ou plusieurs des revendications précédentes, en particulier dans lequel le pansement (62) présente sur la deuxième face extérieure (28) des évidements (84) et/ou bossages (40), dans lequel le pansement (62) présente au moins un évidement (84) dans la zone centrale du pansement (62), de préférence dans lequel un superabsorbant et/ou un indicateur de pH et/ou une substance à efficacité pharmaceutique est disposé(e) dans l'évidement dans la zone centrale du pansement (62).
